# EUROPÄISCHE PATENTSCHRIFT

(11) **EP 0 387 315 B1**
(45) Veröffentlichungstag und Bekanntmachung des Hinweises auf die Patenterteilung: **08.12.1993**
(21) Anmeldenummer: 89907746.5
(22) Anmeldetag: 14.07.1989
(51) Int. Cl.: C07C 69/74, C09K 19/30, C09K 19/20, C09K 19/12, C09K 19/34, C07C 69/773, C07C 43/225, C07D 239/26, C07D 239/34, C07D 319/06

(54) **FLUORIERTE ALKOXYVERBINDUNGEN**
FLUORINATED ALKOXYL COMPOUNDS
COMPOSES ALKOXY FLUORES

(30) Priorität: 27.07.1988 DE 3825424
(43) Veröffentlichungstag der Anmeldung: 19.09.1990
(73) Patentinhaber: MERCK PATENT GmbH, D-64271 Darmstadt (DE)
(72) Erfinder: DORSCH, Dieter, D-6100 Darmstadt (DE); WEBER, Georg, D-6106 Erzhausen (DE); PLACH, Herbert, D-6100 Darmstadt (DE)
(86) Internationale Anmeldenummer: EP8900822
(87) Internationale Veröffentlichungsnummer: WO9001021

(56) Entgegenhaltungen:
- EP-A- 0 255 236
- JP-A-62 129 251
- Journal of Organic Chemistry of the USSR, März 1988, Plenum Publishing Corp., S.V. Shelyazhenko et al.: "Liquid crystals containing fluorine. XI. Influence of electronic effects of terminal groups on mesomorphism in 4'-substituted 4-perfluoro-alkylbiphenyls", Seiten 555-560

## Beschreibung

Die Erfindung betrifft fluorierte Alkoxyverbindungen der Formel I

R-(A¹-Z¹)ₘ-A²-O-CH₂-(CF₂)ₙ-F (I)

worin
- R: H, einen geradkettigen unsubstituierten Alkyl- oder Alkenylrest mit 1 bis 15 C-Atomen, wobei in diesen Resten auch eine oder mehrere CH₂-Gruppen jeweils unabhängig voneinander durch -O-, -CO-, -CO-O-, -O-CO- oder -O-CO-O-so ersetzt sein können, daß O-Atome nicht direkt miteinander verknüpft sind,
- A¹ und A²: jeweils unabhängig voneinander einen
(a) trans-1,4-Cyclohexylenrest, worin auch eine oder mehrere nicht benachbarte CH₂-Gruppen durch -O- und/oder -S- ersetzt sein können,
(b) 1,4-Phenylenrest, worin auch eine oder zwei CH-Gruppen durch N ersetzt sein können,
(c) Rest aus der Gruppe 1,4-Cyclohexenylen, 1,4-Bicyclo(2,2,2)-octylen, Piperidin-1,4-diyl, Naphthalin-2,6-diyl, Decahydronaphthalin-2,6-diyl und 1,2,3,4-Tetrahydronaphthalin-2,6-diyl,
wobei die Reste (a) und (b) durch CN oder Fluor substituiert sein können,
- Z¹: jeweils unabhängig voneinander -CH₂O-, -OCH₂-, -CH₂CH₂-, -CH=CH-, -C≡C- oder eine Einfachbindung,
- m: 1, 2 oder 3 und
- n: 1 bis 16
bedeuten.

Die Erfindung betrifft weiterhin die Verwendung dieser Verbindungen als Komponenten flüssigkristalliner Phasen sowie Flüssigkristall- und elektrooptische Anzeigeelemente, die die erfindungsgemäßen flüssigkristallinen Phasen enthalten.

Aus der EP-A-0 255 236 sind ähnliche Verbindugen bekannt, die jedoch stets eine chirale Flügelgruppe enthalten und somit in ferroelektrischen Mischungen eingesetzt werden.

Aus der Literaturstelle Chemical Abstracts, Vol. 108, 1988, no. 13950h sind Cyclohexansäurephenylester bekannt, die eine ähnliche Flügelgruppe aufweisen, jedoch aufgrund ihrer thermischen Instabilität die hohen Anforderungen, die heutzutage an nematische Mischungen gestellt werden, nicht ganz erfüllen können.

Die Verbindungen der Formel I können als Komponenten flüssigkristalliner Phasen verwendet werden, insbesondere für Displays, die auf dem Prinzip der verdrillten Zelle, dem Guest-Host-Effekt, dem Effekt der Deformation aufgerichteter Phasen oder dem Effekt der dynamischen Streuung beruhen.

Der Erfindung lag die Aufgabe zugrunde, neue stabile flüssigkristalline oder mesogene Verbindungen aufzufinden, die als Komponenten flüssigkristalliner Phasen geeignet sind und insbesondere eine vergleichsweise geringe Viskosität besitzen sowie eine relativ hohe dielektrische Anisotropie.

Es wurde nun gefunden, daß Verbindungen der Formel I als Komponenten flüssigkristalliner Phasen vorzüglich geeignet sind. Insbesondere verfügen sie über vergleichsweise niedere Viskositäten. Mit ihrer Hilfe lassen sich stabile flüssigkristalline Phasen mit breitem Mesophasenbereich und vorteilhaften Werten für die optische und dielektrische Anisotropie erhalten.

Mit der Bereitstellung von Verbindungen der Formel I wird außerdem ganz allgemein die Palette der flüssigkristallinen Substanzen, die sich unter verschiedenen anwendungstechnischen Gesichtspunkten zur Herstellung flüssigkristalliner Gemische eignen, erheblich verbreitert.

Die Verbindungen der Formel I besitzen einen breiten Anwendungsbereich. In Abhängigkeit von der Auswahl der Substituenten können diese Verbindungen als Basismaterialien dienen, aus denen flüssigkristalline Phasen zum überwiegenden Teil zusammengesetzt sind; es können aber auch Verbindungen der Formel I flüssigkristallinen Basismaterialien aus anderen Verbindungsklassen zugesetzt werden, um beispielsweise die dielektrische und/oder optische Anisotropie eines solchen Dielektrikums zu beeinflussen und/oder um dessen Schwellenspannung und/oder dessen Viskosität zu optimieren.

Die Verbindungen der Formel I sind in reinem Zustand farblos und bilden flüssigkristalline Mesophasen in einem für die elektrooptische Verwendung günstig gelegenen Temperaturbereich. Chemisch, thermisch und gegen Licht sind sie stabil.

Gegenstand der Erfindung sind somit die Verbindungen der Formel I sowie die Verwendung dieser Verbindungen als Komponenten flüssigkristalliner Phasen. Gegenstand der Erfindung sind ferner flüssigkristalline Phasen mit einem Gehalt an mindestens einer Verbindung der Formel I sowie Flüssigkristallanzeigeelemente, insbesondere elektroopische Anzeigeelemente, die derartige Phasen enthalten.

Der Einfachheit halber bedeuten im folgenden X O-CH₂-(CF₂)ₙ-F, Cyc einen 1,4-Cyclohexylenrest, Che einen 1,4-Cyclohexenylenrest, Dio einen 1,3-Dioxan-2,5-diylrest, Dit einen 1,3-Dithian-2,5-diylrest, Phe einen 1,4-Phenylenrest, Pyd einen Pyridin-2,5-diylrest, Pyr einen Pyrimidin-2,5-diylrest und Bi einen Bicyclo(2,2,2)-octylenrest, wobei Cyc und/oder Phe unsubstituiert oder ein- oder zweifach durch F oder CN substituiert sein können.

Die Verbindungen der Formel I umfassen dementsprechend Verbindungen mit zwei Ringen der Teilformeln Ia und Ib:

R-A¹-A²-X Ia

R-A¹-Z¹-A²-X Ib

Verbindungen mit drei Ringen der Teilformeln Ic bis If:

R-A¹-A¹-A²-X Ic

R-A¹-Z¹-A¹-Z¹-A²-X Id

R-A¹-Z¹-A¹-A²-X Ie

R-A¹-A¹-Z¹-A²-X If

sowie Verbindungen mit vier Ringen der Teilformeln Ig bis In:

R-A¹-A¹-A¹-A²-X Ig

R-A¹-Z¹-A¹-A¹-A²-X Ih

R-A¹-A¹-Z¹-A¹-A²-X Ii

R-A¹-A¹-A¹-Z¹-A²-X Ij

R-A¹-Z¹-A¹-Z¹-A¹-A²-X Ik

R-A¹-Z¹-A¹-A¹-A¹-Z¹-A²-X Il

R-A¹-A¹-Z¹-A¹-Z¹-A²-X Im

R-A¹-Z¹-A¹-Z¹-A¹-Z¹-A²-X In

Darunter sind besonders diejenigen der Teilformeln Ia, Ib, Ic, Id, Ie, If, Ii und Il bevorzugt.

Die bevorzugten Verbindungen der Teilformel Ia umfassen diejenigen der Teilformeln Iaa bis Iag:

R-Phe-Phe-X Iaa

R-Phe-Cyc-X Iab

R-Dio-Phe-X Iac

R-Pyr-Phe-X Iad

R-Pyd-Phe-X Iae

R-Cyc-Phe-X Iaf

R-Che-Phe-X Iag

Darunter sind diejenigen der Formeln Iaa, Iab, Iac, Iad und Iaf besonders bevorzugt.

Die bevorzugten Verbindungen der Teilformel Ib umfassen diejenigen der Teilformeln Iba bis Ibg:

R-Phe-CH₂CH₂-Phe-X Iba

R-Phe-OCH₂-Phe-X Ibb

R-Cyc-CH₂CH₂-Phe-X Ibc

R-A¹-CH₂-CH₂-Phe-X Ibd

R-A¹-CH₂O-Phe-X Ibe

R-A¹-OCH₂-Phe-X Ibf

R-Che-CH₂CH₂-Phe-X Ibg

Die bevorzugten Verbindungen der Teilformel Ic umfassen diejenigen der Teilformeln Ica bis Icj:

R-Phe-Phe-Phe-X Ica

R-Phe-Phe-Cyc-X Icb

R-Phe-Dio-Phe-X Icc

R-Cyc-Cyc-Phe-X Icd

R-Pyd-Phe-Phe-X Ice

R-Pyr-Phe-Phe-X Icf

R-Cyc-Phe-Phe-X Icg

R-Dio-Phe-Phe-X Ich

R-Che-Phe-Phe-X Ici

R-Phe-Che-Phe-X Icj

Darunter sind diejenigen der Formeln Icd und Icg besonders bevorzugt.

Die bevorzugten Verbindungen der Teilformel Id umfassen diejenigen der Teilformeln Ida bis Idk:

R-Phe-Z¹-Phe-Z¹-Phe-X Ida

R-Phe-Z¹-Phe-Z¹-Cyc-X Idb

R-Phe-Z¹-Dio-Z¹-Phe-X Idc

R-Cyc-Z¹-Cyc-Z¹-Phe-X Idd

R-Pyd-Z¹-Phe-Z¹-Phe-X Ide

R-Phe-Z¹-Pyd-Z¹-Phe-X Idf

R-Pyr-Z¹-Phe-Z¹-Phe-X Idg

R-Phe-Z¹-Pyr-Z¹-Phe-X Idh

R-Phe-Z¹-Cyc-Z¹-Phe-X Idi

R-Dio-Z¹-Phe-Z¹-Phe-X Idj

R-Che-Z¹-Phe-Z¹-Phe-X Idk

Die bevorzugten Verbindungen der Teilformel Ie umfassen diejenigen der Teilformeln Iea bis Iej:

R-Pyr-Z¹-Phe-Phe-X Iea

R-Pyr-Z¹-Phe-Cyc-X Ieb

R-Dio-Z¹-Phe-Phe-X Iec

R-Cyc-Z¹-Phe-Phe-X Ied

R-Phe-Z¹-Cyc-Phe-X Iee

R-Cyc-Z¹-Cyc-Phe-X Ief

R-Phe-Z¹-Dio-Phe-X Ieg

R-Pyd-Z¹-Phe-Phe-X Ieh

R-Phe-Z¹-Pyr-Phe-X Iei

R-Phe-Z¹-Che-Phe-X Iej

Die bevorzugten Verbindungen der Teilformel If umfassen diejenigen der Teilformeln Ifa bis Ifm:

R-Pyr-Phe-Z¹-Phe-X Ifa

R-Pyr-Phe-OCH₂-Phe-X Ifb

R-Phe-Phe-Z¹-Phe-X Ifc

R-Phe-Phe-Z¹-Cyc-X Ifd

R-Cyc-Cyc-Z¹-Phe-X Ife

R-Cyc-Cyc-CH₂CH₂-Phe-X Iff

R-Pyd-Phe-Z¹-Phe-X Ifg

R-Dio-Phe-Z¹-Phe-X Ifh

R-Phe-Cyc-Z¹-Phe-X Ifi

R-Phe-Pyd-Z¹-Phe-X Ifj

R-Che-Phe-Z¹-Phe-X Ifk

R-Phe-Che-Z¹-Phe-X Ifl

R-Cyc-Phe-Z¹-Phe-X Ifm

Die bevorzugten Verbindungen der Teilformeln Ig bis In umfassen diejenigen der Teilformeln Io bis It:

R-Cyc-Cyc-Phe-Phe-X Io

R-A¹-CH₂O-A¹-A¹-Phe-X Ip

R-Cyc-Cyc-Z¹-A¹-Phe-X Iq

R-A¹-A¹-A¹-CH₂CH₂-Phe-X Ir

R-Phe-Z¹-Phe-Z¹-Dio-Phe-X Is

R-Phe-Z¹-Phe-Phe-Z¹-Phe-X It

In den Verbindungen der vor- und nachstehenden Formeln bedeutet X vorzugsweise -O-CH₂-(CF₂)ₙ-F, worin n 1 bis 12, vorzugsweise 1 bis 6 bedeutet. Besonders bevorzugt bedeutet n 1, 2, 3 und 4.

R bedeutet vorzugsweise Alkyl oder Alkenyl, ferner Alkoxy. A¹ bedeutet bevorzugt Phe, Cyc, Che, Pyr oder Dio. Bevorzugt enthalten die Verbindungen der Formel I nicht mehr als einen der Reste Bi, Pyd, Pyr, Dio oder Dit. A² bedeutet bevorzugt Phe, Cyc, Pyr oder Dio. Ganz besonders bevorzugt bedeutet A² Phe und Cyc.

Bevorzugt sind auch Verbindungen der Formel I sowie aller Teilformeln, in denen A¹ und/oder A² ein- oder zweifach durch F oder einfach durch CN substituiertes 1,4-Phenylen bedeutet. Insbesondere sind dies 2-Fluor-1,4-phenylen, 3-Fluor-1,4-phenylen und 2,3-Difluor-1,4-phenylen sowie 2-Cyan-1,4-phenylen und 3-Cyan-1,4-phenylen.

Z¹ bedeutet bevorzugt eine Einfachbindung, oder -CH₂CH₂-, in zweiter Linie bevorzugt -CH₂O-und -OCH₂-.

Falls R einen Alkylrest und/oder einen Alkoxyrest bedeutet, so hat dieser vorzugsweise 2, 3, 4, 5, 6 oder 7 C-Atome und bedeutet demnach bevorzugt Ethyl, Propyl, Butyl, Pentyl, Hexyl, Heptyl, Ethoxy, Propoxy, Butoxy, Pentoxy, Hexoxy oder Heptoxy, ferner Methyl, Octyl, Nonyl, Decyl, Undecyl, Dodecyl, Tridecyl, Tetradecyl, Pentadecyl, Methoxy, Octoxy, Nonoxy, Decoxy, Undecoxy, Dodecoxy, Tridecoxy oder Tetradecoxy.

Oxaalkyl bedeutet vorzugsweise geradkettiges 2-Oxapropyl (= Methoxymethyl), 2- (= Ethoxymethyl) oder 3-Oxabutyl (= 2-Methoxyethyl), 2-, 3- oder 4-Oxapentyl, 2-, 3-, 4- oder 5-Oxahexyl, 2-, 3-, 4-, 5- oder 6-Oxaheptyl, 2-, 3-, 4-, 5-, 6- oder 7-Oxaoctyl, 2-, 3-, 4-, 5-, 6-, 7- oder 8-Oxanonyl, 2-, 3-, 4-, 5-, 6-, 7-, 8- oder 9-Oxadecyl.

Falls R einen Alkylrest bedeutet, in dem eine CH₂-Gruppe durch -CH=CH- ersetzt ist, so hat dieser vorzugsweise 2 bis 10 C-Atome. Er bedeutet demnach besonders Vinyl, Prop-1-, oder Prop-2-enyl, But-1-, 2- oder But-3-enyl, Pent-1-, 2-, 3- oder Pent-4-enyl, Hex-1-, 2-, 3-, 4- oder Hex-5-enyl, Hept-1-, 2-, 3-, 4-, 5- oder Hept-6-enyl, Oct-1-, 2-, 3-, 4-, 5-, 6- oder Oct-7-enyl, Non-1-, 2-, 3-, 4-, 5-, 6-, 7- oder Non-8-enyl, Dec-1-, 2-, 3-, 4-, 5-, 6-, 7-, 8- oder Dec-9-enyl.

Falls R einen Alkylrest bedeutet, in dem eine CH₂-Gruppe durch -O- und eine durch -CO- ersetzt ist, so sind diese bevorzugt benachbart. Somit beeinhalten diese eine Acyloxygruppe -CO-O- oder eine Oxycarbonylgruppe -O-CO-. Vorzugsweise haben diese 2 bis 6 C-Atome. Sie bedeuten demnach besonders Acetyloxy, Propionyloxy, Butyryloxy, Pentanoyloxy, Hexanoyloxy, Acetyloxymethyl, Propionyloxymethyl, Butyryloxymethyl, Pentanoyloxymethyl, 2-Acetyloxyethyl, 2-Propionyloxyethyl, 2-Butyryloxyethyl, 3-Acetyloxypropyl, 3-Propionyloxypropyl, 4-Acetyloxybutyl, Methoxycarbonyl, Ethoxycarbonyl, Propoxycarbonyl, Butoxycarbonyl, Pentoxycarbonyl, Methoxycarbonylmethyl, Ethoxycarbonylmethyl, Propoxycarbonylmethyl, Butoxycarbonylmethyl, 2-(Methoxycarbonyl)ethyl, 2-(Ethoxycarbonyl)ethyl, 2-(Propoxycarbonyl)ethyl, 3-(Methoxycarbonyl)propyl, 3-(Ethoxycarbonyl)propyl, 4-(Methoxycarbonyl)-butyl.

Falls R einen Alkylrest bedeutet, in dem eine CH₂-Gruppe durch -CH=CH- und eine benachbarte CH₂-Gruppe durch CO oder CO-O oder O-CO-ersetzt ist, so hat dieser vorzugsweise 4 bis 13 C-Atome Er bedeutet demnach besonders Acryloyloxymethyl, 2-Acryloyloxyethyl, 3-Acryloyloxypropyl, 4-Acryloyloxybutyl, 5-Acryloyloxypentyl, 6-Acryloyloxyhexyl, 7-Acryloyloxyheptyl, 8-Acryloyloxyoctyl, 9-Acryloyloxynonyl, 10-Acryloyloxydecyl, Methacryloyloxymethyl, 2-Methacryloyloxyethyl, 3-Methacryloyloxypropyl, 4-Methacryloyloxybutyl, 5-Methacryloyloxypentyl, 6-Methacryloyloxyhexyl, 7-Methacryloyloxyheptyl, 8-Methacryloyloxyoctyl, 9-Methacryloyloxynonyl.

Verbindungen der Formel I, die über für Polymerisationsreaktionen geeignete Flügelgruppen R verfügen, eignen sich zur Darstellung flüssigkristalliner Polymerer.

Verbindungen der Formel I, die über für Polykondensationen geeignete Flügelgruppen R verfügen, eignen sich zur Darstellung flüssigkristalliner Polykondensate.

Unter diesen Verbindungen der Formel I sowie den Unterformeln sind diejenigen bevorzugt, in denen mindestens einer der darin enthaltenden Reste eine der angegebenen bevorzugten Bedeutungen hat.

In den Verbindungen der Formel I sind diejenigen Stereoisomeren bevorzugt, in denen die Ringe Cyc und Piperidin trans-1,4-disubstituiert sind. Diejenigen der vorstehend genannten Formeln, die eine oder mehrere Gruppen Pyd, Pyr und/oder Dio enthalten, umschließen jeweils die beiden 2,5-Stellungsisomeren.

Die 1,4-Cyclohexenylen-Gruppe hat vorzugsweise folgende Strukturen: 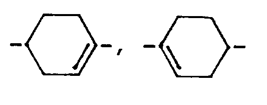
Die Verbindungen der Formel I werden nach an sich bekannten Methoden dargestellt, wie sie in der Literatur (z. B. in den Standardwerken wie Houben-Weyl, Methoden der Organischen Chemie, Georg-Thieme-Verlag, Stuttgart Bd IX, S. 867 ff.) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die erfindungsgemäßen fluorierten Alkoxyverbindungen werden z. B. hergestellt, indem man entsprechende Nitro- oder Fluorbenzole, die mindestens einen elektronenziehenden Substituenten tragen, direkt mit Alkalifluoralkoxyverbindungen umsetzt, wobei F bzw. NO₂ gegen den Fluoralkoxyrest substituiert wird [J.P. Idoux, et al., J. Org. Chem. 50, 1976 (1985)].

Ausgangstoffe können gewünschtenfalls auch in situ gebildet werden, derart, daß man sie aus dem Reaktionsgemisch nicht isoliert, sondern sofort weiter zu den Verbindungen der Formel I umsetzt.

So können die Verbindungen der Formel I hergestellt werden, indem man eine Verbindung, die sonst der Formel I entspricht, aber an Stelle von H-Atomen eine oder mehrere reduzierbare Gruppen und/oder C-C-Bindungen enthält, reduziert.

Als reduzierbare Gruppen kommen vorzugsweise Carbonylgruppen in Betracht, insbesondere Ketogruppen, ferner z.B. freie oder veresterte Hydroxygruppen oder aromatisch gebundene Halogenatome. Bevorzugte Ausgangsstoffe für die Reduktion entsprechen der Formel I, können aber an Stelle eines Cyclohexanringes einen Cyclohexenring oder Cyclohexanonring und/oder an Stelle einer -CH₂CH₂-Gruppe eine -CH=CH-Gruppe und/oder an Stelle einer -CH₂-Gruppe eine -CO-Gruppe und/oder an Stelle eines H-Atoms eine freie oder eine funktionell (z.B. in Form ihres p-Toluolsulfonats) abgewandelte OH-Gruppe enthalten.

Die Reduktion kann z.B. erfolgen durch katalytische Hydrierung bei Temperaturen zwischen etwa O° und etwa 200° sowie Drucken zwischen etwa 1 und 200 bar in einem inerten Lösungsmittel, z.B. einem Alkohol wie Methanol, Ethanol oder Isopropanol, einem Ether wie Tetrahydrofuran (THF) oder Dioxan, einem Ester wie Ethylacetat, einer Carbonsäure wie Essigsäure oder einem Kohlenwasserstoff wie Cyclohexan. Als Katalysatoren eignen sich zweckmäßig Edelmetalle wie Pt oder Pd, die in Form von Oxiden (z.B. PtO₂, PdO), auf einem Träger (z.B. Pd auf Kohle, Calciumcarbonat oder Strontiumcarbonat) oder in feinverteilter Form eingesetzt werden können.

Ketone können auch nach den Methoden von Clemmensen (mit Zink, amalgamiertem Zink oder Zinn und Salzsäure, zweckmäßig in wäßrig-alkoholischer Lösung oder in heterogener Phase mit Wasser/Toluol bei Temperaturen zwischen etwa 80 und 120°) oder Wolff-Kishner (mit Hydrazin, zweckmäßig in Gegenwart von Alkali wie KOH oder NaOH in einem hochsiedenden Lösungsmittel wie Diethylenglykol oder Triethylenglykol bei Temperaturen zwischen etwa 100 und 200°) zu den entsprechenden Verbindungen der Formel I, die Alkylgruppen und/oder -CH₂CH₂-Brücken enthalten, reduziert werden.

Weiterhin sind Reduktionen mit komplexen Hydriden möglich. Beispielsweise können Arylsulfonyloxygruppen mit LiAlH₄ reduktiv entfernt werden, insbesondere p-Toluolsulfonyloxymethylgruppen zu Methylgruppen reduziert werden, zweckmäßig in einem inerten Lösungsmittel wie Diethylether oder THF bei Temperaturen zwischen etwa 0 und 100°. Doppelbindungen können mit NaBH₄ oder Tributylzinnhydrid in Methanol hydriert werden.

Verbindungen der Formel I, die ansonsten der Formel I entsprechen, aber an Stelle von 1,4-Phenylenresten 1,4-Cyclohexenylenreste besitzen, können zum Beispiel mit DDQ (Dichlordicyanobenzochinon) in einem geeigneten Lösungsmittel oxidiert werden.

Ester der Formel I können auch durch Veresterung entsprechender Carbonsäuren (oder ihrer reaktionsfähigen Derivate) mit Alkoholen bzw. Phenolen (oder ihren reaktionsfähigen Derivaten) oder nach der DCC-Methode (DCC = Dicyclohexylcarbodiimid) erhalten werden.

Die entsprechenden Carbonsäuren und Alkohole bzw. Phenole sind bekannt oder können in Analogie zu bekannten Verfahren hergestellt werden.

Als reaktionsfähige Derivate der genannten Carbonsäuren eignen sich insbesondere die Säurehalogenide, vor allem die Chloride und Bromide, ferner die Anhydride, z.B. auch gemischte Anhydride, Azide oder Ester, insbesondere Alkylester mit 1 - 4 C-Atomen in der Alkylgruppe.

Als reaktionsfähige Derivate der genannten Alkohole bzw. Phenole kommen insbesondere die entsprechenden Metallalkoholate bzw. Phenolate, vorzugsweise eines Alkalimetalls wie Natrium oder Kalium, in Betracht.

Die Veresterung wird vorteilhaft in Gegenwart eines inerten Lösungsmittels durchgeführt. Gut geeignet sind insbesondere Ether wie z. B. Diethylether, Di-n-butylether, THF, Dioxan oder Anisol, Ketone wie z. B. Aceton, Butanon oder Cyclohexanon, Amide wie z. B. DMF oder Phosphorsäurehexamethyltriamid, Kohlenwasserstoffe wie z. B. Benzol, Toluol oder Xylol, Halogenkohlenwasserstoffe wie z. B. Tetrachlorkohlenstoff, Dichlormethan oder Tetrachlorethylen und Sulfoxide wie z. B. Dimethylsulfoxid oder Sulfolan.

Ether der Formel I sind durch Veretherung entsprechender Hydroxyverbindungen, vorzugsweise entsprechender Phenole, erhältlich, wobei die Hydroxyverbindung zweckmäßig zunächst in ein entsprechendes Metallderivat, z.B. durch Behandeln mit NaH, NaNH₂, NaOH, KOH, Na₂CO₃ oder K₂CO₃ in das entsprechende Alkalimetallalkoholat oder Alkalimetallphenolat übergeführt wird. Dieses kann dann mit dem entsprechenden Alkylhalogenid, -sulfonat oder Dialkylsulfat umgesetzt werden, zweckmäßig in einem inerten Lösungsmittel wie z. B. Aceton, 1,2-Dimethoxyethan, DMF oder Dimethylsulfoxid oder auch mit einem Überschuß an wäßriger oder wäßrig-alkoholischer NaOH oder KOH bei Temperaturen zwischen etwa 20° und 100°.

Verbindungen der Formel I, worin A¹ durch mindestens ein F-Atom und/oder eine CN-Gruppe substituiert ist, können auch aus den entsprechenden Diazoniumsalzen durch Austausch der Diazoniumgruppe gegen ein Fluoratom oder gegen eine CN-Gruppe, z.B. nach den Methoden von Schiemann oder Sandmeyer, erhalten werden.

Dioxanderivate bzw. Dithiauderivate der Formel I werden zwecksmäßig durch Reaktion eines entsprechenden Aldehyds (oder eines seiner reaktionsfähigen Derivate) mit einem entsprechenden 1,3-Diol (oder einem seiner reaktionsfähigen Derivate) bzw. einem entsprechenden 1,3-Dithiol hergestellt, vorzugsweise in Gegenwart eines inerten Lösungsmittels wie z. B. Benzol oder Toluol und/oder eines Katalysators, z. B. einer starken Säure wie Schwefelsäure, Benzol- oder p-Toluolsulfonsäure, bei Temperaturen zwischen etwa 20° und etwa 150°, vorzugsweise zwischen 80° und 120°. Als reaktionsfähige Derivate der Ausgangsstoffe eignen sich in erster Linie Acetale.

Die genannten Aldehyde und 1,3-Diole bzw. 1,3-Dithiole sowie ihre reaktionsfähigen Derivate sind zum Teil bekannt, zum Teil können sie ohne Schwierigkeiten nach Standardverfahren der Organischen Chemie aus literaturbekannten Verbindungen hergestellt werden. Beispielsweise sind die Aldehyde durch Oxydation entsprechender Alkohole oder durch Reduktion entsprechender Carbonsäuren oder ihrer Derivate, die Diole durch Reduktion entsprechender Diester und die Dithiole durch Umsetzung entsprechender Dihalogenide mit NaSH erhältlich.

Die erfindungsgemäßen flüssigkristallinen Phasen bestehen im allgemeinen aus 2 bis 25, vorzugsweise 3 bis 15 Komponenten, darunter mindestens einer Verbindung der Formel I. Es können in Einzelfällen jedoch auch mehr als 25 Komponenten Bestandteile einer erfindungsgemäßen Phase sein, z. B. bis zu 50 Komponenten und mehr. Die anderen Bestandteile werden vorzugsweise ausgewählt aus den nematischen oder nematogenen Substanzen, insbesondere den bekannten Substanzen, aus den Klassen der Azoxybenzole, Benzylidenaniline, Biphenyle, Terphenyle, Phenyl- oder Cyclohexylbenzoate, Cyclohexan-carbonsäurephenyl- oder -cyclohexyl-ester, Phenylcyclohexane, Cyclohexylbiphenyle, Cyclohexylcyclohexane, Cyclohexylnaphthaline, 1,4-Biscyclohexylbenzole, 4,4'-Biscyclohexylbiphenyle, Phenyl-oder Cyclohexylpyrimidine, Phenyl- oder Cyclohexyldioxane, Phenyl- oder Cyclohexyldithiane, 1,2-Bis-phenylethane, 1,2-Biscyclohexylethane, 1-Phenyl-2-cyclohexylethane, gegebenenfalls halogenierten Stilbene, Benzylphenylether, Tolane und substituierten Zimtsäuren.

Die wichtigsten als Bestandteile erfindungsgemäßer flüssigkristalliner Phasen in Frage kommenden Verbindungen lassen sich durch die Formel II charakterisieren,

R¹-L-G-E-R² II

worin L und E je ein carbo- oder heterocyclisches Ringsystem aus der aus 1,4-disubstituierten Benzol- und Cyclohexanringen, 4,4'-disubstituierten Biphenyl-, Phenylcyclohexan- und Cyclohexylcyclohexansystemen, 2,5-disubstituierten Pyrimidin- und 1,3-Dioxanringen, 2,6-disubstituiertem Naphthalin, Di- und Tetrahydronaphthalin, Chinazolin und Tetrahydrochinazolin gebildeten Gruppe,
- G: -CH=CH- -N(O)=N-
-CH=CY- -CH=N(O)-
-C≡C- -CH₂-CH₂-
-CO-O- -CH₂-O-
-CO-S- -CH₂-S-
-CH=N- -COO-Phe-COO-

oder eine C-C-Einfachbindung, Y Halogen, vorzugsweise Chlor, oder -CN, und R¹ und R² Alkyl, Alkoxy, Alkanoyloxy oder Alkoxycarbonyloxy mit bis zu 18, vorzugsweise bis zu 8 Kohlenstoffatomen, oder einer dieser Reste auch CN, NO₂, CF₃, F, Cl oder Br bedeuten.

Bei den meisten dieser Verbindungen sind R¹ und R² voneinander verschieden, wobei einer dieser Reste meist eine Alkyl- oder Alkoxygruppe ist. Aber auch andere Varianten der vorgesehenen Substituenten sind gebräuchlich. Viele solcher Substanzen oder auch Gemische davon sind im Handel erhältlich. Alle diese Substanzen sind nach literaturbekannten Methoden oder in Analogie dazu herstellbar.

Die erfindungsgemäßen flüssigkristallinen Phasen enthalten etwa 0,1 bis 99, vorzugsweise 10 bis 95 %, einer oder mehrerer Verbindungen der Formel I. Weiterhin bevorzugt sind flüssigkristalline Phasen, die 0,1 - 50, insbesondere 0,5 - 30 % einer oder mehrerer Verbindungen der Formel I enthalten. Auch isotrope Verbindungen der Formel I können in den erfindungsgemäßen Phasen verwendet werden.

Die Herstellung der erfindungsgemäßen flüssigkristallinen Phasen erfolgt in an sich üblicher Weise. In der Regel werden die Komponenten ineinander gelöst, vorzugsweise bei erhöhter Temperatur.

Durch geeignete Zusätze können die flüssigkristallinen Phasen nach der Erfindung so modifiziert werden, daß sie in allen bisher bekannt gewordenen Arten von Flüssigkristallanzeigeelementen verwendet werden können.

Derartige Zusätze sind dem Fachmann bekannt und in der Literatur ausführlich beschrieben. Beispielsweise können Leitsalze, vorzugsweise Ethyl-dimethyl-dodecyl-ammonium-4-hexyloxybenzoat, Tetrabutylammonium-tetraphenylboranat oder Komplexsalze von Kronenethern (vgl. z.B. I. Haller et al., Mol.Cryst.Liq.Cryst. Band 24, Seiten 249 - 258 (1973)) zur Verbesserung der Leitfähigkeit, dichroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden. Derartige Substanzen sind z.B. in den DE-OS 22 09 127, 22 40 864, 23 21 632, 23 38 281, 24 50 088, 26 37 430, 28 53 728 und 29 02 177 beschrieben.

Die folgenden Beispiele sollen die Erfindung erläutern, ohne sie zu begrenzen. Vor- und nachstehend bedeuten Prozentangaben Gewichtsprozent. Alle Temperaturen sind in Grad Celsius angegeben. Fp. bedeutet Schmelzpunkt, Kp = Klärpunkt. Ferner bedeuten K = kristalliner Zustand, N = nematische Phase, S = semektische Phase und I = isotrope Phase. Die Angaben zwischen diesen Symbolen stellen die Übergangstemperaturen dar.

"Übliche Aufarbeitung" bedeutet: man gibt gegebenenfalls Wasser hinzu, extrahiert mit Methylenchlorid, Diethylether oder Toluol, trennt ab, trocknet die organische Phase, dampft ein und reinigt das Produkt durch Destillation unter reduziertem Druck oder Kristallisation und/oder Chromatographie. Folgende Abkürzungen werden verwendet:
- DCC: Dicyclohexylcarbodiimid
- DDQ: Dichlordicyanobenzochinon
- THF: Tetrahydrofuran
- KOT: Kalium-tertiär-butanolat
- pTSOH: p-Toluolsulfonsäure

### Beispiel 1

### 4-{2-[4-(1H,1H-Trifluorethoxy)-phenyl]-ethenyl}-4'-pentyl-trans,trans-bicyclohexyl

Eine Suspension von 0,08 mol 4'-Pentyl-trans,trans-bicyclohexyl-4-methyl-triphenylphosphoniumiodid in 250 ml THF wird unter Eiskühlung mit 0,09 mol 4-(1H, 1H-Trifluorethoxy)-benzaldehyd (vgl. Literaturzitat in Beispiel 1) und 0,08 mol KOT versetzt. Die Mischung wird 1 h bei 5 °C gerührt. Danach wird 2 N HCl bis zur neutralen Reaktion der wäßrigen Phase und Wasser bis zur Lösung des Niederschlags zugegeben. Die organische Phase wird abgetrennt, über Na₂SO₄ getrocknet und eingedampft. Der Rückstand wird über eine Kieselgelsäule mit Petrolether/Ethylacetat 9:1 als Laufmittel chromatographiert. Man erhält farblose Kristalle.

Analog werden hergestellt:
4-{2-[4-(1H,1H-Trifluorethoxy)-phenyl]-ethenyl}-4'-ethyl-trans,trans-bicyclohexyl
4-{2-[4-(1H,1H-Trifluorethoxy)-phenyl]-ethenyl}-4'-propyl-trans,trans-bicyclohexyl
4-{2-[4-(1H,1H-Trifluorethoxy)-phenyl]-ethenyl}-4'-butyl-trans,trans-bicyclohexyl
4-{2-[4-(1H,1H-Trifluorethoxy)-phenyl]-ethenyl}-4'-hexyl-trans,trans-bicyclohexyl
4-{2-[4-(1H,1H-Trifluorethoxy)-phenyl]-ethenyl}-4'-heptyl-trans,trans-bicyclohexyl
4-{2-[4-(1H,1H-Trifluorethoxy)-phenyl]-ethenyl}-4'-octyl-trans,trans-bicyclohexyl
4-{2-[4-(1H,1H-Pentafluorpropoxy)-phenyl]-ethenyl}-4'-ethyl-trans,trans-bicyclohexyl
4-{2-[4-(1H,1H-Pentafluorpropoxy)-phenyl]-ethenyl}-4'-propyl-trans,trans-bicyclohexyl
4-{2-[4-(1H,1H-Pentafluorpropoxy)-phenyl]-ethenyl}-4'-butyl-trans,trans-bicyclohexyl
4-{2-[4-(1H,1H-Pentafluorpropoxy)-phenyl]-ethenyl}-4'-pentyl-trans,trans-bicyclohexyl
4-{2-[4-(1H,1H-Pentafluorpropoxy)-phenyl]-ethenyl}-4'-hexyl-trans,trans-bicyclohexyl
4-{2-[4-(1H,1H-Pentafluorpropoxy)-phenyl]-ethenyl}-4'-heptyl-trans,trans-bicyclohexyl
4-{2-[4-(1H,1H-Pentafluorpropoxy)-phenyl]-ethenyl}-4'-octyl-trans,trans-bicyclohexyl
4-{2-[4-(1H,1H-Heptafluorbutoxy)-phenyl]-ethenyl}-4'-ethyl-trans,trans-bicyclohexyl
4-{2-[4-(1H,1H-Heptafluorbutoxy)-phenyl]-ethenyl}-4'-propyl-trans,trans-bicyclohexyl
4-{2-[4-(1H,1H-Heptafluorbutoxy)-phenyl]-ethenyl}-4'-butyl-trans,trans-bicyclohexyl
4-{2-[4-(1H,1H-Heptafluorbutoxy)-phenyl]-ethenyl}-4'-pentyl-trans,trans-bicyclohexyl
4-{2-[4-(1H,1H-Heptafluorbutoxy)-phenyl]-ethenyl}-4'-hexyl-trans,trans-bicyclohexyl
4-{2-[4-(1H,1H-Heptafluorbutoxy)-phenyl]-ethenyl}-4'-heptyl-trans,trans-bicyclohexyl
4-{2-[4-(1H,1H-Heptafluorbutoxy)-phenyl]-ethenyl}-4'-octyl-trans,trans-bicyclohexyl

### Beispiel 2

### 4-{2-[4-(1H,1H-Trifluorethoxy)-phenyl]-ethyl}-4'-pentyl-trans,trans-bicyclohexyl

Eine Lösung von 0,038 mol des Produkts 3 in 150 ml Essigester wird mit 10 g Pd/C (5 % Pd) versetzt und bei Raumtemperatur und Normaldruck hydriert. Der Katalysator wird abfiltriert, das Filtrat eingedampft und der Rückstand aus Ethanol umkristallisiert. Man erhält farblose Kristalle. K 66 S 177 I
Analog werden hergestellt:
4-{2-[4-(1H,1H-Trifluorethoxy)-phenyl]-ethyl}-4'-ethyl-trans,trans-bicyclohexyl
4-{2-[4-(1H,1H-Trifluorethoxy)-phenyl]-ethyl}-4'-propyl-trans,trans-bicyclohexyl
4-{2-[4-(1H,1H-Trifluorethoxy)-phenyl]-ethyl}-4'-butyl-trans,trans-bicyclohexyl
4-{2-[4-(1H,1H-Trifluorethoxy)-phenyl]-ethyl}-4'-hexyl-trans,trans-bicyclohexyl
4-{2-[4-(1H,1H-Trifluorethoxy)-phenyl]-ethyl}-4'-heptyl-trans,trans-bicyclohexyl
4-{2-[4-(1H,1H-Trifluorethoxy)-phenyl]-ethyl}-4'-octyl-trans,trans-bicyclohexyl
4-{2-[4-(1H,1H-Pentafluorpropoxy)-phenyl]-ethyl}-4'-ethyl-trans,trans-bicyclohexyl
4-{2-[4-(1H,1H-Pentafluorpropoxy)-phenyl]-ethyl}-4'-propyl-trans,trans-bicyclohexyl
4-{2-[4-(1H,1H-Pentafluorpropoxy)-phenyl]-ethyl}-4'-butyl-trans,trans-bicyclohexyl
4-{2-[4-(1H,1H-Pentafluorpropoxy)-phenyl]-ethyl}-4'-pentyl-trans,trans-bicyclohexyl
4-{2-[4-(1H,1H-Pentafluorpropoxy)-phenyl]-ethyl}-4'-hexyl-trans,trans-bicyclohexyl
4-{2-[4-(1H,1H-Pentafluorpropoxy)-phenyl]-ethyl}-4'-heptyl-trans,trans-bicyclohexyl
4-{2-[4-(1H,1H-Pentafluorpropoxy)-phenyl]-ethyl}-4'-octyl-trans,trans-bicyclohexyl
4-{2-[4-(1H,1H-Heptafluorbutoxy)-phenyl]-ethyl}-4'-ethyl-trans,trans-bicyclohexyl
4-{2-[4-(1H,1H-Heptafluorbutoxy)-phenyl]-ethyl}-4'-propyl-trans,trans-bicyclohexyl
4-{2-[4-(1H,1H-Heptafluorbutoxy)-phenyl]-ethenyl}-4'-butyl-trans,trans-bicyclohexyl
4-{2-[4-(1H,1H-Heptafluorbutoxy)-phenyl]-ethenyl}-4'-pentyl-trans,trans-bicyclohexyl
4-{2-[4-(1H,1H-Heptafluorbutoxy)-phenyl]-ethenyl}-4'-hexyl-trans,trans-bicyclohexyl
4-{2-[4-(1H,1H-Heptafluorbutoxy)-phenyl]-ethenyl}-4'-heptyl-trans,trans-bicyclohexyl
4-{2-[4-(1H,1H-Heptafluorbutoxy)-phenyl]-ethenyl}-4'-octyl-trans,trans-bicyclohexyl

### Beispiel 3

### 1H,1H-Trifluorethoxy-4-(5-ethyl-1,3-dioxan-2-yl)-benzol

0,1 mol 4-(1H,1H-Trifluorethoxy)-benzaldehyd, 0,1 mol Ethylpropandiol und 0,2 g p-TsOH werden in 100 ml Toluol 2 Stunden zum Sieden erhitzt. Nach Eindampfen des Lösungsmittels wird unter reduziertem Druck destilliert. Man erhält farblose Kristalle.

Analog werden hergestellt:
1H,1H-Trifluorethoxy-4-(5-propyl-1,3-dioxan-2-yl)-benzol
1H,1H-Trifluorethoxy-4-(5-butyl-1,3-dioxan-2-yl)-benzol
1H,1H-Trifluorethoxy-4-(5-pentyl-1,3-dioxan-2-yl)-benzol
1H,1H-Trifluorethoxy-4-(5-hexyl-1,3-dioxan-2-yl)-benzol
1H,1H-Trifluorethoxy-4-(5-heptyl-1,3-dioxan-2-yl)-benzol
1H,1H-Trifluorethoxy-4-(5-octyl-1,3-dioxan-2-yl)-benzol
1H,1H-Trifluorethoxy-4-(5-nonyl-1,3-dioxan-2-yl)-benzol
1H,1H-Trifluorethoxy-4-(5-decyl-1,3-dioxan-2-yl)-benzol
1H,1H-Trifluorethoxy-4-(5-methoxy-1,3-dioxan-2-yl)-benzol
1H,1H-Trifluorethoxy-4-(5-ethoxy-1,3-dioxan-2-yl)-benzol
1H,1H-Trifluorethoxy-4-(5-propoxy-1,3-dioxan-2-yl)-benzol
1H,1H-Trifluorethoxy-4-(5-butoxy-1,3-dioxan-2-yl)-benzol
1H,1H-Trifluorethoxy-4-(5-pentoxy-1,3-dioxan-2-yl)-benzol
1H,1H-Trifluorethoxy-4-(5-hexoxy-1,3-dioxan-2-yl)-benzol
1H,1H-Trifluorethoxy-4-(5-heptoxy-1,3-dioxan-2-yl)-benzol
1H,1H-Trifluorethoxy-4-(5-octoxy-1,3-dioxan-2-yl)-benzol
1H,1H-Trifluorethoxy-4-(5-nonoxy-1,3-dioxan-2-yl)-benzol
1H,1H-Pentafluorpropoxy-4-(5-propyl-1,3-dioxan-2-yl)-benzol
1H,1H-Pentafluorpropoxy-4-(5-ethyl-1,3-dioxan-2-yl)-benzol
1H,1H-Pentafluorpropoxy-4-(5-butyl-1,3-dioxan-2-yl)-benzol
1H,1H-Pentafluorpropoxy-4-(5-pentyl-1,3-dioxan-2-yl)-benzol
1H,1H-Pentafluorpropoxy-4-(5-hexyl-1,3-dioxan-2-yl)-benzol
1H,1H-Pentafluorpropoxy-4-(5-heptyl-1,3-dioxan-2-yl)-benzol
1H,1H-Pentafluorpropoxy-4-(5-octyl-1,3-dioxan-2-yl)-benzol
1H,1H-Pentafluorpropoxy-4-(5-nonyl-1,3-dioxan-2-yl)-benzol
1H,1H-Pentafluorpropoxy-4-(5-decyl-1,3-dioxan-2-yl)-benzol
1H,1H-Pentafluorpropoxy-4-(5-methoxy-1,3-dioxan-2-yl)-benzol
1H,1H-Pentafluorpropoxy-4-(5-ethoxy-1,3-dioxan-2-yl)-benzol
1H,1H-Pentafluorpropoxy-4-(5-propoxy-1,3-dioxan-2-yl)-benzol
1H,1H-Pentafluorpropoxy-4-(5-butoxy-1,3-dioxan-2-yl)-benzol
1H,1H-Pentafluorpropoxy-4-(5-pentoxy-1,3-dioxan-2-yl)-benzol
1H,1H-Pentafluorpropoxy-4-(5-hexoxy-1,3-dioxan-2-yl)-benzol
1H,1H-Pentafluorpropoxy-4-(5-heptoxy-1,3-dioxan-2-yl)-benzol
1H,1H-Pentafluorpropoxy-4-(5-octoxy-1,3-dioxan-2-yl)-benzol
1H,1H-Pentafluorpropoxy-4-(5-nonoxy-1,3-dioxan-2-yl)-benzol
1H,1H-Heptafluorbutoxy-4-(5-propyl-1,3-dioxan-2-yl)-benzol
1H,1H-Heptafluorbutoxy-4-(5-ethyl-1,3-dioxan-2-yl)-benzol
1H,1H-Heptafluorbutoxy-4-(5-butyl-1,3-dioxan-2-yl)-benzol
1H,1H-Heptafluorbutoxy-4-(5-pentyl-1,3-dioxan-2-yl)-benzol
1H,1H-Heptafluorbutoxy-4-(5-hexyl-1,3-dioxan-2-yl)-benzol
1H,1H-Heptafluorbutoxy-4-(5-heptyl-1,3-dioxan-2-yl)-benzol
1H,1H-Heptafluorbutoxy-4-(5-octyl-1,3-dioxan-2-yl)-benzol
1H,1H-Heptafluorbutoxy-4-(5-nonyl-1,3-dioxan-2-yl)-benzol
1H,1H-Heptafluorbutoxy-4-(5-decyl-1,3-dioxan-2-yl)-benzol
1H,1H-Heptafluorbutoxy-4-(5-methoxy-1,3-dioxan-2-yl)-benzol
1H,1H-Heptafluorbutoxy-4-(5-ethoxy-1,3-dioxan-2-yl)-benzol
1H,1H-Heptafluorbutoxy-4-(5-propoxy-1,3-dioxan-2-yl)-benzol
1H,1H-Heptafluorbutoxy-4-(5-butoxy-1,3-dioxan-2-yl)-benzol
1H,1H-Heptafluorbutoxy-4-(5-pentoxy-1,3-dioxan-2-yl)-benzol
1H,1H-Heptafluorbutoxy-4-(5-hexoxy-1,3-dioxan-2-yl)-benzol
1H,1H-Heptafluorbutoxy-4-(5-heptoxy-1,3-dioxan-2-yl)-benzol
1H,1H-Heptafluorbutoxy-4-(5-octoxy-1,3-dioxan-2-yl)-benzol
1H,1H-Heptafluorbutoxy-4-(5-nonoxy-1,3-dioxan-2-yl)-benzol

### Beispiel 4

### 1H,1H-Trifluorethoxy-4-(5-heptyl-1,3-pyrimidin-2-yl)-benzol

Ein Gemisch aus 0,1 mol p-1H,1H-Trifluorethoxy-benzimidamid-hydrochlorid (erhältlich aus dem Nitril (vgl. Literaturzitat in Beispiel 1) über das entsprechende Benzimidsäureethylester-hydrochlorid) und 0,1 mol Heptylmalondialdehydbisdiethylacetal wird 15 h bei 150 °C gerührt. Nach Abkühlen wird wie üblich aufgearbeitet.

Analog werden hergestellt
1H,1H-Trifluorethoxy-4-(5-ethyl-1,3-pyrimidin-2-yl)-benzol
1H,1H-Trifluorethoxy-4-(5-propyl-1,3-pyrimidin-2-yl)-benzol
1H,1H-Trifluorethoxy-4-(5-butyl-1,3-pyrimidin-2-yl)-benzol
1H,1H-Trifluorethoxy-4-(5-pentyl-1,3-pyrimidin-2-yl)-benzol
1H,1H-Trifluorethoxy-4-(5-hexyl-1,3-pyrimidin-2-yl)-benzol
1H,1H-Trifluorethoxy-4-(5-methoxy-1,3-pyrimidin-2-yl)-benzol
1H,1H-Trifluorethoxy-4-(5-propoxy-1,3-pyrimidin-2-yl)-benzol
1H,1H-Trifluorethoxy-4-(5-butoxy-1,3-pyrimidin-2-yl)-benzol
1H,1H-Trifluorethoxy-4-(5-pentoxy-1,3-pyrimidin-2-yl)-benzol
1H,1H-Trifluorethoxy-4-(5-hexoxy-1,3-pyrimidin-2-yl)-benzol
1H,1H-Trifluorethoxy-4-(5-heptoxy-1,3-pyrimidin-2-yl)-benzol
1H,1H-Pentafluorpropoxy-4-(5-ethyl-1,3-pyrimidin-2-yl)-benzol
1H,1H-Pentafluorpropoxy-4-(5-propyl-1,3-pyrimidin-2-yl)-benzol
1H,1H-Pentafluorpropoxy-4-(5-butyl-1,3-pyrimidin-2-yl)-benzol
1H,1H-Pentafluorpropoxy-4-(5-pentyl-1,3-pyrimidin-2-yl)-benzol
1H,1H-Pentafluorpropoxy-4-(5-hexyl-1,3-pyrimidin-2-yl)-benzol
1H,1H-Pentafluorpropoxy-4-(5-heptyl-1,3-pyrimidin-2-yl)-benzol
1H,1H-Pentafluorpropoxy-4-(5-methoxy-1,3-pyrimidin-2-yl)-benzol
1H,1H-Pentafluorpropoxy-4-(5-propoxy-1,3-pyrimidin-2-yl)-benzol
1H,1H-Pentafluorpropoxy-4-(5-butoxy-1,3-pyrimidin-2-yl)-benzol
1H,1H-Pentafluorpropoxy-4-(5-pentoxy-1,3-pyrimidin-2-yl)-benzol
1H,1H-Pentafluorpropoxy-4-(5-hexoxy-1,3-pyrimidin-2-yl)-benzol
1H,1H-Pentafluorpropoxy-4-(5-heptoxy-1,3-pyrimidin-2-yl)-benzol

### Beispiel 5

### 4-(4-Propyl-cyclohexen-1-yl)-1H,1H-trifluorethoxybenzol

In eine Suspension von 0,12 mol Magnesiumspänen in 50 ml wasserfreiem THF wird eine Lösung von 0,1 mol 4-Brom-1H,1H-Trifluorethoxybenzol [erhältlich aus der 4-Nitroverbindung durch Reduktion, und anschließender Sandmeyer-Reaktion (vgl. Organikum VEB Deutscher Verlag der Wissenschaften, Berlin 1977, S. 662-665)] bei Raumtemperatur in 50 ml wasserfreiem THF zugetropft. Anschließend wird eine Lösung von 0,1 mol 4-Propylcyclohexanon in 100 ml THF zugetropft, dann 2 h bei Raumtemperatur, abschließend 2 h in der Siedehitze gerührt. Das abgekühlte Gemisch wird in kalte verdünnte HCl (2 N) gegossen und das Produkt mit Diethylether extrahiert. Nach dem Abziehen des Solvens wird der Rückstand in 150 ml Toluol gelöst und nach Zugabe von 0,006 mol p-TsOH 4 h zum Sieden erhitzt. Es wird wie üblich aufgearbeitet.

Analog werden hergestellt:
4-(4-Ethyl-cyclohexen-1-yl)-1H,1H-trifluorethoxybenzol
4-(4-Butyl-cyclohexen-1-yl)-1H,1H-trifluorethoxybenzol
4-(4-Pentyl-cyclohexen-1-yl)-1H,1H-trifluorethoxybenzol
4-(4-Hexyl-cyclohexen-1-yl)-1H,1H-trifluorethoxybenzol
4-(4-Heptyl-cyclohexen-1-yl)-1H,1H-trifluorethoxybenzol
4-(4-Octyl-cyclohexen-1-yl)-1H,1H-trifluorethoxybenzol
4-(4-Ethyl-cyclohexen-1-yl)-1H,1H-pentafluorpropoxybenzol
4-(4-Propyl-cyclohexen-1-yl)-1H,1H-pentafluorpropoxybenzol
4-(4-Butyl-cyclohexen-1-yl)-1H,1H-pentafluorpropoxybenzol
4-(4-Pentyl-cyclohexen-1-yl)-1H,1H-pentafluorpropoxybenzol
4-(4-Hexyl-cyclohexen-1-yl)-1H,1H-pentafluorpropoxybenzol
4-(4-Heptyl-cyclohexen-1-yl)-1H,1H-pentafluorpropoxybenzol
4-(4-Octyl-cyclohexen-1-yl)-1H,1H-pentafluorpropoxybenzol
4-(4-Ethyl-cyclohexen-1-yl)-1H,1H-heptafluorbutoxybenzol
4-(4-Propyl-cyclohexen-1-yl)-1H,1H-heptafluorbutoxybenzol
4-(4-Butyl-cyclohexen-1-yl)-1H,1H-heptafluorbutoxybenzol
4-(4-Pentyl-cyclohexen-1-yl)-1H,1H-heptafluorbutoxybenzol
4-(4-Hexyl-cyclohexen-1-yl)-1H,1H-heptafluorbutoxybenzol
4-(4-Heptyl-cyclohexen-1-yl)-1H,1H-heptafluorbutoxybenzol
4-(4-Octyl-cyclohexen-1-yl)-1H,1H-heptafluorbutoxybenzol

### Beispiel 6

### 4-(trans-4-Propylcyclohexyl)-1H,1H-trifluorethoxybenzol

In eine mit 5 g Pd/C suspendierte Lösung von 0,13 mol des Produkts aus Beispiel 6 in 100 ml THF wird bei Raumtemperatur bis zur Sättigung Wasserstoff eingeleitet. Nach 3 h Rühren wird wie üblich aufgearbeitet.

Analog werden hergestellt
4-(trans-4-Ethylcyclohexyl)-1H,1H-trifluorethoxybenzol
4-(trans-4-Butylcyclohexyl)-1H,1H-trifluorethoxybenzol
4-(trans-4-Pentylcyclohexyl)-1H,1H-trifluorethoxybenzol
4-(trans-4-Hexylcyclohexyl)-1H,1H-trifluorethoxybenzol
4-(trans-4-Heptylcyclohexyl)-1H,1H-trifluorethoxybenzol
4-(trans-4-Octylcyclohexyl)-1H,1H-trifluorethoxybenzol
4-(trans-4-Ethylcyclohexyl)-1H,1H-pentafluorpropoxybenzol
4-(trans-4-Propylcyclohexyl)-1H,1H-pentafluorpropoxybenzol
4-(trans-4-Butylcyclohexyl)-1H,1H-pentafluorpropoxybenzol
4-(trans-4-Pentylcyclohexyl)-1H,1H-pentafluorpropoxybenzol
4-(trans-4-Hexylcyclohexyl)-1H,1H-pentafluorpropoxybenzol
4-(trans-4-Heptylcyclohexyl)-1H,1H-pentafluorpropoxybenzol
4-(trans-4-Octylcyclohexyl)-1H,1H-pentafluorpropoxybenzol
4-(trans-4-Ethylcyclohexyl)-1H,1H-nonafluorpentoxybenzol
4-(trans-4-Propylcyclohexyl)-1H,1H-nonafluorpentoxybenzol
4-(trans-4-Butylcyclohexyl)-1H,1H-nonafluorpentoxybenzol
4-(trans-4-Pentylcyclohexyl)-1H,1H-nonafluorpentoxybenzol
4-(trans-4-Hexylcyclohexyl)-1H,1H-nonafluorpentoxybenzol
4-(trans-4-Heptylcyclohexyl)-1H,1H-nonafluorpentoxybenzol
4-(trans-4-Octylcyclohexyl)-1H,1H-nonafluorpentoxybenzol
4-[trans-4-(trans-4-Ethylcyclohexyl)-cyclohexyl]-1H,1H,-trifluorethoxybenzol
4-[trans-4-(trans-4-Propylcyclohexyl)-cyclohexyl]-1H,1H,-trifluorethoxybenzol
4-[trans-4-(trans-4-Butylcyclohexyl)-cyclohexyl]-1H,1H,-trifluorethoxybenzol
4-[trans-4-(trans-4-Pentylcyclohexyl)-cyclohexyl]-1H,1H,-trifluorethoxybenzol
4-[trans-4-(trans-4-Hexylcyclohexyl)-cyclohexyl]-1H,1H,-trifluorethoxybenzol
4-[trans-4-(trans-4-Octylcyclohexyl)-cyclohexyl]-1H,1H,-trifluorethoxybenzol
4-[trans-4-(trans-4-Ethylcyclohexyl)-cyclohexyl]-1H,1H,-pentafluorpropoxybenzol
4-[trans-4-(trans-4-Propylcyclohexyl)-cyclohexyl]-1H,1H,-pentafluorpropoxybenzol
4-[trans-4-(trans-4-Butylcyclohexyl)-cyclohexyl]-1H,1H,-pentafluorpropoxybenzol
4-[trans-4-(trans-4-Pentylcyclohexyl)-cyclohexyl]-1H,1H,-pentafluorpropoxybenzol
4-[trans-4-(trans-4-Hexylcyclohexyl)-cyclohexyl]-1H,1H,-pentafluorpropoxybenzol
4-[trans-4-(trans-4-Octylcyclohexyl)-cyclohexyl]-1H,1H,-pentafluorpropoxybenzol
4-[trans-4-(trans-4-Ethylcyclohexyl)-cyclohexyl]-1H,1H,-heptafluorbutoxybenzol
4-[trans-4-(trans-4-Propylcyclohexyl)-cyclohexyl]-1H,1H,-heptafluorbutoxybenzol
4-[trans-4-(trans-4-Butylcyclohexyl)-cyclohexyl]-1H,1H,-heptafluorbutoxybenzol
4-[trans-4-(trans-4-Pentylcyclohexyl)-cyclohexyl]-1H,1H,-heptafluorbutoxybenzol
4-[trans-4-(trans-4-Hexylcyclohexyl)-cyclohexyl]-1H,1H,-heptafluorbutoxybenzol
4-[trans-4-(trans-4-Octylcyclohexyl)-cyclohexyl]-1H,1H,-heptafluorbutoxybenzol

### Beispiel 7

### 4-Propyl-4'-1H,1H-trifluorethoxy-1,1'-biphenyl

Eine Lösung von 0,1 mol des Produkts aus Beispiel 7 und 0,2 mol DDQ in 800 ml Toluol wird 2 h zum Sieden erhitzt. Nach dem Abkühlen wird wie üblich aufgearbeitet.

Analog werden hergestellt:
4-Ethyl-4'-1H,1H-trifluorethoxy-1,1'-biphenyl
4-Propyl-4'-1H,1H-trifluorethoxy-1,1'-biphenyl
4-Butyl-4'-1H,1H-trifluorethoxy-1,1'-biphenyl
4-Pentyl-4'-1H,1H-trifluorethoxy-1,1'-biphenyl
Fp: 107 °C
4-Hexyl-4'-1H,1H-trifluorethoxy-1,1'-biphenyl
4-Heptyl-4'-1H,1H-trifluorethoxy-1,1'-biphenyl
4-Octyl-4'-1H,1H-trifluorethoxy-1,1'-biphenyl
4-Methoxy-4'-1H,1H-trifluorethoxy-1,1'-biphenyl
4-Ethoxy-4'-1H,1H-trifluorethoxy-1,1'-biphenyl
4-Propoxy-4'-1H,1H-trifluorethoxy-1,1'-biphenyl
4-Butoxy-4'-1H,1H-trifluorethoxy-1,1'-biphenyl
4-Pentoxy-4'-1H,1H-trifluorethoxy-1,1'-biphenyl
4-Heptoxy-4'-1H,1H-trifluorethoxy-1,1'-biphenyl
4-Nonoxy-4'-1H,1H-trifluorethoxy-1,1'-biphenyl
4-Ethyl-4'-1H,1H-pentafluorpropoxy-1,1'-biphenyl
4-Propyl-4'-1H,1H-pentafluorpropoxy-1,1'-biphenyl
4-Butyl-4'-1H,1H-pentafluorpropoxy-1,1'-biphenyl
4-Pentyl-4'-1H,1H-pentafluorpropoxy-1,1'-biphenyl
4-Hexyl-4'-1H,1H-pentafluorpropoxy-1,1'-biphenyl
4-Heptyl-4'-1H,1H-pentafluorpropoxy-1,1'-biphenyl
4-Octyl-4'-1H,1H-pentafluorpropoxy-1,1'-biphenyl
4-Methoxy-4'-1H,1H-pentafluorpropoxy-1,1'-biphenyl
4-Ethoxy-4'-1H,1H-pentafluorpropoxy-1,1'-biphenyl
4-Propoxy-4'-1H,1H-pentafluorpropoxy-1,1'-biphenyl
4-Butoxy-4'-1H,1H-pentafluorpropoxy-1,1'-biphenyl
4-Pentoxy-4'-1H,1H-pentafluorpropoxy-1,1'-biphenyl
4-Heptoxy-4'-1H,1H-pentafluorpropoxy-1,1'-biphenyl
4-Nonoxy-4'-1H,1H-pentafluorpropoxy-1,1'-biphenyl
4-Ethyl-4'-1H,1H-heptafluorbutoxy-1,1'-biphenyl
4-Propyl-4'-1H,1H-heptafluorbutoxy-1,1'-biphenyl
4-Butyl-4'-1H,1H-heptafluorbutoxy-1,1'-biphenyl
4-Pentyl-4'-1H,1H-heptafluorbutoxy-1,1'-biphenyl
4-Hexyl-4'-1H,1H-heptafluorbutoxy-1,1'-biphenyl
4-Heptyl-4'-1H,1H-Heptafluorbutoxy-1,1'-biphenyl
4-Octyl-4'-1H,1H-Heptafluorbutoxy-1,1'-biphenyl
4-Methoxy-4'-1H,1H-Heptafluorbutoxy-1,1'-biphenyl
4-Ethoxy-4'-1H,1H-Heptafluorbutoxy-1,1'-biphenyl
4-Propoxy-4'-1H,1H-Heptafluorbutoxy-1,1'-biphenyl
4-Butoxy-4'-1H,1H-Heptafluorbutoxy-1,1'-biphenyl
4-Pentoxy-4'-1H,1H-Heptafluorbutoxy-1,1'-biphenyl
4-Heptoxy-4'-1H,1H-Heptafluorbutoxy-1,1'-biphenyl
4-Nonoxy-4'-1H,1H-Heptafluorbutoxy-1,1'-biphenyl
4-(trans-4-Ethylcyclohexyl)-1H,1H-trifluorethoxy-1,1'-biphenyl
4-(trans-4-Propylcyclohexyl)-1H,1H-trifluorethoxy-1,1'-biphenyl
4-(trans-4-Butylcyclohexyl)-1H,1H-trifluorethoxy-1,1'-biphenyl
4-(trans-4-Heptylcyclohexyl)-1H,1H-heptafluorbutoxy-1,1'-biphenyl
4-(trans-4-Octylcyclohexyl)-1H,1H-heptafluorbutoxy-1,1'-biphenyl
Folgendes Beispiel betrifft eine erfindungsgemäße flüssigkristalline Phase.

Eine flüssigkristalline Phase, bestehend aus
8 % 4-{2-[4-(1H,1H-Trifluorethoxy)-phenyl]-ethyl}-4'-propyl-trans,trans-bicyclohexyl
8 % 4-{2-[4-(1H,1H-Trifluorethoxy)-phenyl]-ethyl}-4'-pentyl-trans,trans-bicyclohexyl
8 % 4-{2-[4-(1H,1H-Trifluorethoxy)-phenyl]-ethyl}-4'-heptyl-trans,trans-bicyclohexyl
15 % trans-1-p-Methoxyphenyl-4-propylcyclohexan
15 % trans-1-p-Ethoxyphenyl-4-propylcyclohexan
14 % trans-1-p-Butoxyphenyl-4-propylcyclohexan
5 % 4-{2-[4-Methylphenyl]-ethyl}-4'-propyl-trans, trans-bicyclohexyl
5 % 4-{2-[4-Ethylphenyl]-ethyl}-4'-propyl-trans, trans-bicyclohexyl
5 % 4-{2-[4-Propylphenyl]-ethyl}-4'-propyl-trans, trans-bicyclohexyl
5 % 4-{2-[4-Pentylphenyl]-ethyl}-4'-propyl-trans, trans-bicyclohexyl
4 % 4-(trans-4-Propylcyclohexyl-2'-fluor-4'-(trans-4-propylcyclohexyl)-biphenyl
4 % 2-Fluor-4-(trans-4-pentylcyclohexyl)-4'-(trans-4-propylcyclohexyl)-biphenyl
und
4 % 4-(trans-4-Pentylcyclohexyl)-2'-fluor-4'-(trans-4-pentylcyclohexyl)-biphenyl
hat einen Schmelzpunkt von 11,9 °C, einen Klärpunkt von 94 °C und eine Viskosität von 16 mm²/s bei 20 °C.

## Patentansprüche

1. Fluorierte Alkoxyverbindungen der Formel I,
R-(A¹-Z¹)ₘ-A²-O-CH₂-(CF₂)ₙ-F I
worin
R H, einen geradkettigen, unsubstituierten Alkyl- oder Alkenylrest mit 1 bis 15 C-Atomen, wobei in diesen Resten auch eine oder mehrere CH₂-Gruppen jeweils unabhängig voneinander durch -O-, -CO-, -CO-O-, -O-CO- oder -O-CO-O- so ersetzt sein können, daß O-Atome nicht direkt miteinander verknüpft sind,
A¹ und A² jeweils unabhängig voneinander einen
(a) trans-1,4-Cyclohexylenrest, worin auch eine oder mehrere nicht benachbarte CH₂-Gruppen durch -O- und/oder -S- ersetzt sein können,
(b) 1,4-Phenylenrest, worin auch eine oder zwei CH-Gruppen durch N ersetzt sein können,
(c) Rest aus der Gruppe 1,4-Cyclohexenylen, 1,4-Bicyclo(2,2,2)-octylen, Piperidin-1,4-diyl, Naphthalin-2,6-diyl, Decahydronaphthalin-2,6-diyl und 1,2,3,4-Tetrahydronaphthalin-2,6-diyl,
wobei die Reste (a) und (b) durch CN oder Fluor substituiert sein können,
Z¹ jeweils unabhängig voneinander -CH₂O-, -OCH₂-, -CH₂CH₂-, -CH=CH-, -C≡C- oder eine Einfachbindung,
m 1, 2 oder 3 und
n 1 bis 16
bedeuten.

2. Fluorierte Alkoxyverbindungen der Formel I nach Anspruch 1, worin A² ein- oder zweifach durch F substituiertes 1,4-Phenylen bedeutet.

3. Fluorierte Alkoxyverbindungen der Formel I nach Anspruch 2, worin A¹ ein- oder zwei Fach durch F substituiertes 1,4-Phenylen bedeutet.

4. Fluorierte Alkoxyverbindungen nach Anspruch 1, die den Formeln
R-Cyc-Z¹-Phe-Phe-X Ied
R-Cyc-Z¹-Cyc-Z¹-Phe-X Idd
R-Cyc-Phe-Z¹-Phe-X Ifm
entsprechen, worin R und Z¹ die in Anspruch 1 gegebene Bedeutung haben, Cyc tras-1,4-Cyclohexylen, Phe unsubstituiertes oder ein- oder zweifach durch F substituiertes 1,4-Phenylen und X -O-CH₂-(CF₂)ₙ-F mit n = 1 - 16 bedeutet.

5. Fluorierte Alkoxyverbindungen nach Anspruch 1, die der Formel
R-A¹-CH₂CH₂-Phe-X Ibd
entsprechen, worin R und A¹ die in Anspruch 1 gegebene Bedeutung haben, Phe unsubstituiertes oder ein- oder zweifach durch F substituiertes 1,4-Phenylen und X -O-CH₂-(CF₂)ₙ-F mit n = 1 - 16 bedeutet.

6. Fluorierte Alkoxyverbindungen nach Anspruch 1, die den Formeln
R-Phe-Phe-X Iaa
R-Phe-Cyc-X Iab
R-Dio-Phe-X Iac
R-Pyr-Phe-X Iad
R-Cyc-Phe-X Iaf
entsprechen, worin R die in Anspruch 1 gegebene Bedeutung hat, Cyc trans-1,4-Cyclohexylen, Dio 1,3-Dioxan-2,5-diyl, Pyr Pyrimidin-2,5-diyl, Phe unsubstituiertes oder ein- oder zweifach durch F substituiertes 1,4-Phenylen und X -O-CH₂-(CF₂)ₙ-F mit n = 1 - 16 bedeutet.

7. Fluorierte Alkoxyverbindungen nach mindestens einem der Ansprüche 1 - 6, worin in der Gruppe -O-CH₂(CF₂)ₙ-F n = 1 bedeutet.

8. Verwendung von Verbindungen der Formel I als Komponenten flüssigkristalliner Phasen.

9. Flüssigkristalline Phase mit mindestens zwei flüssigkristallinen Komponenten, dadurch gekennzeichnet, daß sie mindestens eine Verbindung der Formel I enthält.

10. Flüssigkristall-Anzeigeelement, dadurch gekennzeichnet, daß es als Dielektrikum eine flüssigkristalline Phase nach Anspruch 9 enthält.

11. Elektrooptisches Anzeigeelement, dadurch gekennzeichnet, daß es als Dielektrikum eine flüssigkristalline Phase nach Anspruch 9 enthält.

## Claims

1. Fluorinated alkoxy compounds of the formula I
R-(A¹-Z¹)ₘ-A²-O-CH₂-(CF₂)ₙ-F I
in which
R is H or a straight-chain, unsubstituted alkyl or alkenyl radical having 1 to 15 carbon atoms, it also being possible for one or more CH₂ groups in these radicals, in each case independently of one another, to be replaced by -O-, -CO-, -CO-O-, -O-CO- or -O-CO-O- in such a way that O atoms are not linked directly to one another,
A¹ and A² are each, independently of one another, a
(a) trans-1,4-cyclohexylene radical in which, in addition, one or more non-adjacent CH₂ groups may be replaced by -O- and/or -S-,
(b) 1,4-phenylene radical in which, in addition, one or two CH groups may be replaced by N,
(c) radical from the group consisting of 1,4-cyclohexenylene, 1,4-bicyclo(2,2,2)octylene, piperidine-1,4-diyl, naphthalene-2,6-diyl, decahydronaphthalene-2,6-diyl and 1,2,3,4-tetrahydronaphthalene-2,6-diyl,
where the radicals (a) and (b) may be substituted by CN or fluorine,
Z¹ are in each case, independently of one another, -CH₂O-, -OCH₂-, -CH₂CH₂-, -CH=CH-, -C≡C- or a single bond,
m is 1, 2 or 3, and
n is 1 to 16.

2. Fluorinated alkoxy compounds of the formula I according to Claim 1, in which A² is 1,4-phenylene which is monosubstituted or disubstituted by F.

3. Fluorinated alkoxy compounds of the formula I according to Claim 2, in which A¹ is 1,4-phenylene which is monosubstituted or disubstituted by F.

4. Fluorinated alkoxy compounds according to Claim 1, which conform to the formulae
R-Cyc-Z¹-Phe-Phe-X Ied
R-Cyc-Z¹-Cyc-Z¹-Phe-X Idd
R-Cyc-Phe-Z¹-Phe-X Ifm
in which R and Z¹ are as defined in Claim 1, Cyc is trans-1,4-cyclohexylene, Phe is 1,4-phenylene which is unsubstituted or monosubstituted or disubstituted by F, and X is -O-CH₂-(CF₂)ₙ-F, where n = 1 - 16.

5. Fluorinated alkoxy compounds according to Claim 1, which conform to the formula
R-A¹-CH₂CH₂-Phe-X Ibd
in which R and A¹ are as defined in Claim 1, Phe is 1,4-phenylene which is unsubstituted or monosubstituted or disubstituted by F, and X is -O-CH₂-(CF₂)ₙ-F, where n = 1 - 16.

6. Fluorinated alkoxy compounds according to Claim 1, which conform to the formulae
R-Phe-Phe-X Iaa
R-Phe-Cyc-X Iab
R-Dio-Phe-X Iac
R-Pyr-Phe-X Iad
R-Cyc-Phe-X Iaf
in which R is as defined in Claim 1, Cyc is trans-1,4-cyclohexylene, Dio is 1,3-dioxane-2,5-diyl, Pyr is pyrimidine-2,5-diyl, Phe is 1,4-phenylene which is unsubstituted or monosubstituted or disubstituted by F, and X is -O-CH₂-(CF₂)ₙ-F, where n = 1 - 16.

7. Fluorinated alkoxy compounds according to at least one of Claims 1 - 6, in which n = 1 in the -O-CH₂-(CF₂)ₙ-F group.

8. Use of compounds of the formula I as components of liquid-crystalline phases.

9. Liquid-crystalline phase having at least two liquid-crystalline components, characterized in that it contains at least one compound of the formula I.

10. Liquid-crystal display element, characterized in that it contains, as dielectric, a liquid-crystalline phase according to Claim 9.

11. Electro-optical display element, characterized in that it contains, as dielectric, a liquid-crystalline phase according to Claim 9.

## Revendications

1. Dérivés alcoxylés fluorés de formule I
R-(A¹-Z¹)ₘ-A²-O-CH₂-(CF₂)ₙ-F I
dans laquelle
R représente un groupe alkyle ou alcényle en C1-C15 à chaîne droite non substitué dans lequel un ou plusieurs groupes CH₂ peuvent être remplacés chacun, indépendamment les uns des autres, par -O-, -CO-, -CO-O-, -O-CO- ou -O-CO-O- en sorte qu'il n'y ait pas de liaison directe entre des atomes d'oxygène,
A¹ et A² représentent chacun, indépendamment l'un de l'autre,
(a) un groupe trans-1,4-cyclohexylène dans lequel un ou plusieurs groupes CH₂ non voisins peuvent être remplacés par -O- et/ou -S-,
(b) un groupe 1,4-phénylène dans lequel un ou deux groupes CH peuvent être remplacés par N,
(c) un groupe choisi parmi les groupes 1,4-cyclohexénylène, 1,4-bicyclo(2,2,2)-octylène, pipéridine-1,4-diyle, naphtalène-2,6-diyle, décahydronaphtalène-2,6-diyle et 1,2,3,4-tétrahydronaphtalène-2,6-diyle,
les groupes (a) et (b) pouvant être substitués par CN ou le fluor,
chacun des symboles Z¹ représente, indépendamment, -CH₂O-, -OCH₂-, -CH₂CH₂-, -CH=CH-, -C≡C- ou une liaison simple,
m est égal à 1, 2 ou 3 et
n est un nombre allant de 1 à 16.

2. Dérivés alcoxylés fluorés de formule I selon revendication 1 dans laquelle A² représente un groupe 1,4-phénylène mono- ou di-substitué par F.

3. Dérivés alcoxylés fluorés de formule I selon revendication 2, dans laquelle A¹ représente un groupe 1,4-phénylène mono- ou disubstitué par F.

4. Dérivés alcoxylés fluorés selon revendication 1, répondant aux formules
R-Cyc-Z¹-Phe-Phe-X Ied
R-Cyc-Z¹-Cyc-Z¹-Phe-X Idd
R-Cyc-Phe-Z¹-Phe-X Ifm
dans lesquelles R et Z¹ ont les significations indiquées dans la revendication 1, Cyc représente un groupe trans-1,4-cyclohexylène, Phe représente un groupe 1,4-phénylène non substitué ou mono- ou disubstitué par F, et X représente -O-CH₂-(CF₂)ₙ-F avec n = 1 à 16.

5. Dérivés alcoxylés fluorés selon revendication 1, répondant à la formule
R-A¹-CH₂CH₂-Phe-X Ibd
dans laquelle R et A ont les significations indiquées dans la revendication 1, Phe représente un groupe 1,4-phénylène non substitué ou mono- ou disubstitué par F et X représente -O-CH₂-(CF₂)ₙ-F avec n = 1 à 16.

6. Dérivés alcoxylés fluorés selon revendication 1, répondant aux formules
R-Phe-Phe-X Iaa
R-Phe-Cyc-X Iab
R-Dio-Phe-X Iac
R-Pyr-Phe-X Iad
R-Cyc-Phe-X Iaf
dans lesquelles R a les significations indiquées dans la revendication 1, Cyc représente un groupe trans-1,4-cyclohexylène, Dio représente un groupe 1,3-dioxanne-2,5-diyle, Pyr représente un groupe pyrimidine-2,5-diyle, Phe représente un groupe 1,4-phénylène non substitué ou mono- ou disubstitué par F et X représente -O-CH₂-(CF₂)ₙ-F avec n = 1 à 16.

7. Dérivés alcoxylés fluorés selon au moins une des revendications 1 à 6, pour lesquels, dans le groupe -O-CH₂-(CF₂)ₙ-F, n = 1.

8. Utilisation des composés de formule I en tant que composants de phases à cristaux liquides.

9. Phase à cristaux liquides à au moins deux composants à cristaux liquides, caractérisée en ce qu'elle contient au moins un composé de formule I.

10. Elément d'affichage à cristaux liquides, caractérisé en ce qu'il contient en tant que diélectrique une phase à cristaux liquides selon revendication 9.

11. Elément d'affichage électro-optique, caractérisé en ce qu'il contient en tant que diélectrique une phase à cristaux liquides selon revendication 9.
